# EUROPEAN PATENT APPLICATION

(11) **EP 4 400 091 A1**
(43) Date of publication of application: **17.07.2024**
(21) Application number: 23151605.5
(22) Date of filing: 13.01.2023
(51) Int. Cl.: A61K 8/49, A61Q 19/08

(54) **COMPOSITIONS COMPRISING MYRICITRIN FOR TREATING SIGNS OF AGING**

(71) Applicant: DSM IP Assets B.V., 6411 TE Heerlen (NL)
(72) Inventor: BUDEL, Leithe, 4303 Kaiseraugst (CH)
(74) Representative: dsm-firmenich IP

(57) **Abstract**

The present invention relates to the (cosmetic) use of myricitrin for treating the signs of skin and/ or hair aging.

## Description

The present invention relates to the (cosmetic) use of myricitrin for treating the signs of skin and/ or hair aging.

Life expectancy is constantly increasing in many countries around the globe. Accordingly, the demand for treatments counteracting or even reversing aging processes is constantly growing, not only for age-related diseases but also for dealing with the physical signs of aging. Thus, it is not surprising that the need for cosmetic products that may be applied topically on skin to combat the signs of skin aging, such as wrinkles and age spots, has been growing significantly lately.

Aging of the skin and hair is a complex process. Intrinsic aging is an inevitable physiological process that results in thin, dry skin, fine wrinkles, decreased elasticity, aberrant pigmentation, hair greying, and hair loss. Extrinsic aging is caused by external environment factors notably solar radiation and air pollution and results in coarse wrinkles, loss of elasticity, laxity, and rough-textured appearance. Other factors that can further contribute to an aged appearance include general poor health, an unhealthy diet, cigarette smoking, and alcohol.

Today, it is well established that cell senescence plays an important role in aging processes. Cell senescence is a physiological process and a tumour-suppressive cell fate characterized by a permanent and irreversible cell cycle arrest as well as acquisition of a pro-inflammatory and proteolytic secretome. Cells that become senescent grow in size and cease to replicate. In addition, senescent cells generate a potent mix of molecules known as the senescence-associated secretory phenotype (SASP), which provokes the immune system into an inflammatory state, disrupts tissue structure and function, and in turn encourages nearby cells to also become senescent. The SASP is one of the key characteristics that distinguish senescent cells from quiescent, terminally differentiated, and other types of non-proliferating cells.

Intrinsic aging is typically correlated with an increase of senescent cells, including senescent skin cells. Senescence of dermal fibroblasts for example is considered a key driver of the aging-related phenotype in skin. Senescence of hair follicle dermal papilla cells (HFDPCs) on the other hand is considered a key driver of hair loss and baldness. Hence, there is an ongoing need to find agents which are able to selectively get rid of these errant cells.

Senolytics, which are typically understood as agents able to selectively kill senescent cells, were the first potential senotherapy to be successfully tested in preclinical in vivo models. In other words, an agent is considered as having senolytic activity when its activity includes specifically inducing or promoting cell death, in particular apoptotic cell death, of senescent cells in the respective tissue. Several senolytic agents have been identified by now such as navitoclax. Said senolytic agents are able to selectively kill senescent cells by induction of apoptosis process, but not on non-senescent cells or normal cells.

A drawback of currently known senolytic agents is that they are often of synthetic origin, have rather complex chemical structures that require significant efforts to be obtained, exhibit low solubility in conventional cosmetic oils, and/ or may have severe undesired side effects.

Therefore, there is an ongoing need for senolytic agents of natural origin which are, for example, obtainable in an economic manner from plant-based sources.

Surprisingly, it has now been found that myricitrin (Synonym: myricetin-3-O-rhamnoside, compound of formula (I)) exhibits a senolytic activity on human dermal fibroblasts as well as on hair follicle dermal papilla cells (HFDPCs) and can thus be used to treat the signs of skin as well as hair aging.

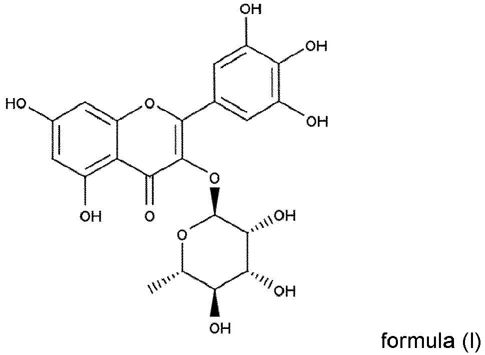

Accordingly, in a first embodiment, the present invention relates to the cosmetic use of myricitrin as an active ingredient for the treatment of the signs of skin and/ or hair aging.

In another embodiment, the invention also provides the use of myricitrin as an active ingredient in, and for the manufacture of, cosmetic compositions for treating signs of skin and/ or hair aging.

In a further embodiment, the present invention also provides myricitrin as an active ingredient for use in the selective elimination of senescent (skin and/ or hair) cells.

The invention also provides the use of myricitrin as an active ingredient in, and for the manufacture of, cosmetic compositions for the selective elimination of senescent (skin and/ or hair) cells.

The invention also provides a cosmetic method for treating signs of skin and/ or hair aging, comprising topically applying to an external surface of the human body a cosmetic composition containing myricitrin.

The present invention also refers to a cosmetic skin and/ or hair care method for selective removal of senescent cells in the skin preferably of senescent human dermal fibroblasts and/ or hair follicle dermal papilla cells by applying, onto the skin (including the scalp and/ or hair) in need, at least an effective amount of an extract of myricitrin; or a composition comprising myricitrin and optionally appreciating the effect. Preferably, the effective amount of myricitrin (based on dry matter) applied on said skin or hair cells is selected in the range of 0.001 to 1 wt.-%/cm² of skin.

As used herein, the term 'cosmetic' refers to a treatment which does not cure, treat, or prevent a disease or disorder, but instead serves as a skincare or hair care product intended to beautify or improve the appearance of the skin or hair, e.g., the colour or texture of the skin or hair.

In the context of this invention, the term 'treating' denotes reducing, preventing, or eliminating.

The expression 'signs of skin aging' as used herein denotes one or more characteristics of intrinsic or chronological skin aging such as thin skin, fine wrinkles, decreased elasticity and aberrant pigmentation. These signs affect everyone, whatever their skin type or state of health. As noted above, the process may be amplified by extrinsic factors such as exposure to sunlight, pollutants, and cigarette smoke.

Accordingly, the term 'treating the signs of skin aging' includes the prevention, reduction or treatment of fine lines, wrinkles, crow's feet, sagging, skin thinning, age spots as well as improving skin elasticity or skin firmness without being limited thereto. The term 'prevention, reducing or treating skin aging' also encompass smoothening of wrinkles and fine lines as well as decreasing their volume and depth in a person in need thereof.

The expression 'signs of hair aging' as used herein denotes one or more characteristics of intrinsic or chronological hair aging such as hair loss and baldness. As noted above, the process may be amplified by extrinsic factors such as exposure to sunlight, pollutants, and cigarette smoke. Accordingly, the term 'treating the signs of hair aging' includes the prevention, reduction or treatment of hair loss and baldness. The term also includes stimulating hair growth as well as retaining hair.

It is well understood in all embodiments of the present invention that the term 'skin' respectively the term 'the external surface of the human body' includes all of the skin of a human body including oral cavities as well as the scalp. Preferably, in all embodiments of the present invention the skin respectively the external surface of the human body treated according to the present invention is the scalp, the face, neck and/or body skin, most preferably the face (including the (lateral) cheek, forehead, nose, chin) skin or the scalp.

The term 'skin cells' as used herein may refer to any skin resident cell type (such as dermal fibroblasts, melanocytes, and epidermal keratinocytes). Preferably in all embodiments of the present invention, the term refers to (human) dermal fibroblasts.

The term 'hair cells' as used herein may refer to any hair follicle resident cell type (such as hair follicle dermal papilla cells). Preferably in all embodiments of the present invention, the term refers to (human) hair follicle dermal papilla cells (HFDPCs).

In the context of this invention, the term 'senescent' refers to a state of permanent cell cycle arrest in which cells remain metabolically active and adopt characteristic phenotypic changes. The establishment of this phenotype is believed to be either the result of telomere shortening after several cell divisions (replicative senescence) or a response to stress stimuli (stress-induced senescence). One of the defining features of senescent cells is their stable cell cycle arrest. This cell cycle exit is controlled by activation of the p53/p21 and p16^{INK4a}/Rb tumour suppressor pathways. Unlike quiescent cells, senescent cells are nonresponsive to mitogenic or growth factor stimuli; thus, they are unable to re-enter the cell cycle even in advantageous growth conditions. Senescent cells are also distinct from terminally differentiated cells, which are also irreversibly withdrawn from the cell cycle. While terminal differentiation is the result of a defined developmental programme, which turns undifferentiated precursors into specialized effector cells, senescence is mainly implemented as a cellular stress response.

Senescent cells can unambiguously be identified in vitro and in vivo since they exhibit a number of characteristics that allow their explicit identification.

For example, senescent cells often appear multinucleated, large and extended, and exhibit spindle and vacuolisation features. They also display modifications in the organisation of chromatin that can help identify them. In normal cells, DNA staining reveals completely uniform colour outlines, whereas senescent cells usually show dot-like patterns, known as senescence-associated heterochromatic foci (SAHF). This phenomenon is due to intensive remodelling in the chromatin, which results in less susceptibility for digestion by nucleases.

Furthermore, senescence-related chromatin remodelling leads to profound transcriptional changes. Among the assortment of upregulated genes is a prominent subset of genes that encode secreted proteins, including cytokines and chemokines with proinflammatory properties, as well as various growth factors and proteases that together alter tissue structure and function, collectively known as SASP. The SASP is one of the key characteristics that distinguish senescent cells from quiescent, terminally differentiated, and other types of non-proliferating cells.

A distinctive measurable feature of senescent cells is the presence of β-galactosidase enzymatic activity. This enzyme normally displays activity at pH 4.0 within lysosomes, but in senescent cells it is also active at pH 6.0. This phenomenon is termed senescence associated-β-galactosidase (SA- β-gal) activity and is thought to be due to an enlargement in the structure of lysosomes in senescent cells. SA-β-gal activity is detectable by histochemical staining by using X-gal as a substrate for SA-β-gal. Since SA-β-gal activity is detected in most senescent settings, both in vitro and in vivo, it is considered a de facto hallmark of senescence. SA-β-gal can be visualized in the cells to quantify the senescence levels using flow cytometry.

The term 'senolytic activity' as used herein refers to specifically (also: selectively) inducing or promoting cell death of senescent cells. This may also be considered as ability for eliminating selectively senescent cells, for example, by inducing or promoting apoptosis. It may also be the capability to selectively eliminate senescent cells found in a tissue with a reduced or even no harmful effect on the normal resident (i.e., non-senescent) cells of the said tissue. Such senolytic property may be beneficial, for example, for preventing or attenuating the increase of the number of age-related senescent cells and/or stress-related senescent cells within a tissue.

Selective elimination of senescent cells may be determined by the change in ratio of senescent (decreasing) versus proliferating cells (increasing) based on SA-β-gal activity in the cell population model after incubation with a fixed concentration of a test compound in a suitable medium as illustrated in the examples. Such a cell population model may contain a set percentage ratio of senescent versus proliferating cells. By comparing with and without added test compound conditions, senolytic activity can be evaluated.

Compounds may be determined as capable of selectively eliminating senescent cells if the calculated senescent cell ratio (based on SA-β-gal activity) is reduced in a statistically significant manner versus the control (no compound added during incubation).

In one preferred embodiment, preferably the number of senescent cells is reduced by at least 10%, preferably by at least 15%, more preferably by at least 20%, most preferably by at least 30% such as by at least 50 %, based on the initial number of senescent cells present. It is well understood, that concomitantly, the number of normal cells is increased by the same factor.

In another embodiment, preferably, the number of senescent cells is reduced by at least a statistical difference in percentage between the control and treated conditions, more preferably by at least 50% in difference (half of original senescent cells removed), most preferably by at least 90% in difference (90% of original senescent cells removed), based on the initial number of senescent cells present. It is well understood, that concomitantly, the number of normal cells is increased by the same factor.

In all embodiments of the present invention the myricitrin is preferably administered in the form of a cosmetic composition comprising an effective amount of the myricitrin and a physiologically acceptable carrier.

The term 'an effective amount' refers to an amount necessary to obtain the desired physiological effect. The physiological effect may be achieved by one application dose or by repeated applications. The dosage administered may, of course, vary depending upon known factors, such as the physiological characteristics of the particular composition comprising the myricitrin and its mode and route of administration; the age, health and weight of the recipient; the kind of concurrent treatment; the frequency of treatment; and the effect desired and can be adjusted by a person skilled in the art.

The term "physiologically acceptable carrier" means a carrier that is compatible with an administration to a human subject, suited to the desired administration route of the composition i.e., in the case of oral administration, a medium that is compatible with the digestive system and in the case of topical administration compatible with the skin, and/or mucous membranes, and compatible with the form in which the composition is intended to be packaged, in particular solid or fluid at ambient temperature and atmospheric pressure. Such carriers are well-known to one of ordinary skill in the art and can include one or more compatible liquid(s) or solid filler diluent(s), excipient(s), additive(s) or vehicle(s) which are suitable for oral or topical application.

Generally, the amount of the myricitrin in the cosmetic compositions according to the present invention is selected in the range from 0.001 to 10 wt.-%, more preferably from 0.01 to 8 wt.-%, even more preferably from 0.1 to 7 wt.-%, furthermore preferably from 0.1 to 5 wt.-% and still more preferably from 0.1 to 3 wt.-%, based on the total weight of the cosmetic composition. Further suitable ranges are from 0.05 to 2 wt.-% and from 0.1 to 1 wt.-%. Further preferred ranges are from 0.001 to 0.5 wt.-%, 0.001 to 1 wt.-%, 0.01 to 1 wt.-% as well as from 0.01 to 0.5 wt.-%,

The exact amount of carrier will depend upon the actual level of the myricitrin and of any other optional ingredients that one of ordinary skill in the art would classify as distinct from the carrier (e.g., other active ingredients).

The cosmetic compositions according to the present invention are preferably prepared by admixing the myricitrin with all the definitions and preferences as given herein with/into a physiologically, preferably cosmetically acceptable carrier.

The cosmetic compositions of the invention (including the carrier) may comprise further conventional (cosmetic) adjuvants and additives, such as preservatives/antioxidants, fatty substances/oils, water, organic solvents, silicones, thickeners, softeners, emulsifiers, antifoaming agents, aesthetic components such as fragrances, aroma ingredients, surfactants, fillers, anionic, cationic, non-ionic or amphoteric polymers or mixtures thereof, propellants, acidifying or basifying agents, dyes, colourings/colorants, abrasives, absorbents, chelating agents and/or sequestering agents, essential oils, skin sensates, astringents, pigments or any other ingredients usually formulated into such compositions.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

In all embodiments of the present invention, the cosmetic compositions according to the present invention are topical cosmetic compositions, i.e., compositions intended to be applied topically to the skin or the scalp.

In an advantageous embodiment, the topical cosmetic compositions according to the present invention comprise from about 50% to about 99%, preferably from about 60% to about 98%, more preferably from about 70% to about 98%, such as in particular from about 80% to about 95% of a carrier, based on the total weight of the composition.

In a particular advantageous embodiment, the carrier of such topical cosmetic compositions consists furthermore of at least 40 wt.-%, more preferably of at least 50 wt.-%, most preferably of at least 55 wt.-% of water, such as in particular of about 55 to about 90 wt.-% of water.

The topical cosmetic compositions according to the present invention may comprise further ingredients, which may form part of the carrier. Such ingredients are particularly surfactants, emulsifiers, thickeners, and oils. Such suitable surfactants, emulsifiers, thickeners, and oils are well known to a person skilled in the art.

In accordance with the present invention, the topical cosmetic compositions according to the invention may also comprise further cosmetically active ingredients conventionally used in topical cosmetic composition. Exemplary active ingredients encompass skin lightening agents; UV-filters, agents for the treatment of hyperpigmentation; agents for the prevention or reduction of inflammation; firming, moisturizing, soothing, and/or energizing agents as well as agents to improve elasticity and skin barrier.

If nothing else is stated, the excipients, additives, diluents, etc. mentioned in the following are suitable for the topical cosmetic compositions according to the present invention. The necessary amounts of the cosmetic and dermatological adjuvants and additives can, based on the desired product, easily be determined by the skilled person.

The additional ingredients can either be added to the oily phase, the aqueous phase or separately as deemed appropriate. The mode of addition can easily be adapted by a person skilled in the art.

Examples of cosmetic excipients, diluents, adjuvants, additives as well as active ingredients commonly used in the skin care industry which are suitable for use in the cosmetic compositions for topical application of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The cosmetically active ingredients useful herein can in some instances provide more than one benefit or operate via more than one mode of action.

The topical cosmetic compositions according to the present invention can be skin care preparations or hair care preparations or both (e.g. hair and body shampoo).

The skin care preparations according to the present invention are in particular skin care preparations, or functional (anti-aging) preparations.

Examples of skin care preparations are, in particular, light protective preparations (sun care preparations), anti-aging preparations, preparations for the treatment of photo-aging, body oils, body lotions, body gels, treatment creams, skin protection ointments, moisturizing preparations such as moisturizing gels or moisturizing sprays, face and/or body moisturizers, as well as skin lightening preparations.

Examples of functional preparations are cosmetic composition containing active ingredients such as hormone preparations, vitamin preparations, vegetable extract preparations, anti-aging preparations, and/or antimicrobial (antibacterial or antifungal) preparations without being limited thereto.

The topical cosmetic composition according to the present invention can be in a wide variety of forms. Non limiting examples include simple solutions (e.g., aqueous, organic solvent, or oil based), emulsion or micro emulsion (in particular of oil-in-water (O/W) or water-in-oil (W/O) type, silicone-in-water (Si/W) or water-in-silicone (W/Si) type, PIT-emulsion, multiple emulsion (e.g., of oil-in-water-in oil (O/W/O)) or water-in-oil-in-water (W/O/W) type) or pickering emulsions), as well as solid forms (e.g., hydrogels, alcoholic gels, lipogels, sticks, flowable solids, or amorphous materials).

These product forms may be used for a number of applications, including, but not limited to, gels, creams, ointments, lotions, serums, powder, aerosol sprays or two component dispensing systems.

In a preferred embodiment, the topical cosmetic composition according to the present invention are emulsions and/or gels. Even more preferably, the topical cosmetic composition are emulsions which contain an oily phase and an aqueous phase such as in particular O/W, W/O, Si/W, W/Si, O/W/O, W/O/W multiple or a pickering emulsions.

The amount of the oily phase (i.e., the phase containing all oils and fats including the polar oils) present in such emulsions such as in particular O/W, W/O, Si/W, W/Si, O/W/O, W/O/W multiple or a pickering emulsions is preferably at least 10 wt.-%, such as in the range from 10 to 60 wt.-%, preferably in the range from 15 to 50 wt.-%, most preferably in the range from 15 to 40 wt.-%, based on the total weight of the composition.

The oil phase according to the invention preferably comprises oils selected from butylenglykoldicaprylat/-dicaprat, propylenglykoldicaprylat/-dicaprat, dicaprylylether, C12-15-Alkylbenzoat, C18 38 fatty acid triglyceride, dibutyladipate, cyclomethicone, dimethicone, 2-phenylethylbenzoat, isopropyl lauroyl sarkosinate, caprylic/capric triglyceride as well as mixtures thereof.

The amount of the aqueous phase present in such emulsions is preferably at least 20 wt.-%, such as in the range from 20 to 90 wt.-%, preferably in the range from 30 to 80 wt.-%, most preferably in the range from 30 to 70 wt.-%, based on the total weight of the composition.

Advantageously in all emulsions of the present invention the ratio of oily phase to aqueous phase is selected in the range of 40:60 to 30:70.

In one particular advantageous embodiment, the topical cosmetic compositions according to the present invention are in the form of an oil-in-water (O/W) emulsion comprising an oily phase dispersed in an aqueous phase in the presence of an O/W emulsifier. The preparation of such O/W emulsions is well known to a person skilled in the art. Preferably such compositions are skin care compositions.

If the topical cosmetic (preferably skin care) composition according to the invention is an O/W emulsion, then it contains advantageously at least one O/W- or Si/W-emulsifier selected from the list of, glyceryl stearate citrate, glyceryl stearate SE (self-emulsifying), stearic acid, salts of stearic acid, polyglyceryl-3-methylglycosedistearate. Further suitable emulsifiers are phosphate esters and the salts thereof such as cetyl phosphate (e.g., as Amphisol^{®} A from DSM Nutritional Products Ltd.), diethanolamine cetyl phosphate (e.g., as Amphisol^{®} DEA from DSM Nutritional Products Ltd.), potassium cetyl phosphate (e.g., as Amphisol^{®} K from DSM Nutritional Products Ltd.), sodium cetearylsulfate, sodium glyceryl oleate phosphate, hydrogenated vegetable glycerides phosphate and mixtures thereof. Further suitable emulsifiers are sorbitan oleate, sorbitan sesquioleate, sorbitan isostearate, sorbitan trioleate, cetearyl glucoside, lauryl glucoside, decyl glucoside, sodium stearoyl glutamate, sucrose polystearate and hydrated polyisobutene. Furthermore, one or more synthetic polymers may be used as an emulsifier. For example, PVP eicosene copolymer, acrylates/C 10-30 alkyl acrylate crosspolymer, and mixtures thereof.

The at least one O/W, respectively Si/W emulsifier is preferably used in an amount of 0.5 to 10 wt. %, in particular in the range of 0.5 to 6 wt.-%, such as more in particular in the range of 0.5 to 5 wt.-%, such as most in particular in the range of 1 to 4 wt.-%, based on the total weight of the composition.

Particular suitable O/W emulsifiers to be used in the topical cosmetic (preferably skincare) composition according to the invention encompass phosphate ester emulsifiers such as advantageously 8-10 alkyl ethyl phosphate, C9-15 alkyl phosphate, ceteareth-2 phosphate, ceteareth-5 phosphate, ceteth-8 phosphate, ceteth-10 phosphate, cetyl phosphate, C6-10 pareth-4 phosphate, C12-15 pareth-2 phosphate, C12-15 pareth-3 phosphate, DEA-ceteareth-2 phosphate, DEA-cetyl phosphate, DEA-oleth-3 phosphate, potassium cetyl phosphate, deceth-4 phosphate, deceth-6 phosphate and trilaureth-4 phosphate.

A particular suitable O/W emulsifier to be used in the topical cosmetic (preferably skincare) composition according to the invention is potassium cetyl phosphate e.g., commercially available as Amphisol^{®} K at DSM Nutritional Products Ltd Kaiseraugst.

Another particular suitable class of O/W emulsifiers are non-ionic self-emulsifying systems derived from olive oil e.g., known as (INCI Name) cetearyl olivate and sorbitan olivate (chemical composition: sorbitan ester and cetearyl ester of olive oil fatty acids) sold under the tradename OLIVEM 1000.

In one particular embodiment, the invention relates to topical cosmetic (preferably skincare) composition with all the definitions and preferences given herein in the form of O/W emulsions comprising an oily phase dispersed in an aqueous phase in the presence of an O/W emulsifier wherein the O/W emulsifier is potassium cetyl phosphate. The amount of oily phase in such O/W emulsions is preferably at least 10 wt.-%, more preferably in the range of 10 to 60 wt.-%, most preferably in the range of 15 to 50 wt.-%, such as in the range of 15 to 40 wt.-%, based on the total weight of the composition.

Preferably, the topical cosmetic (preferably skincare) composition according to the invention further comprise at least one fatty alcohol (co-emulsifier), such as in particular cetyl alcohol, cetearyl alcohol and/or behenyl alcohol. The total amount of one or several fatty alcohols on the cosmetic compositions according to the invention is preferably selected in the range of about 0.1 to 10.0 wt.-%, in particular in the range of about 0.5 to 6.0 wt.-% with respect to the total weight of the composition.

Preferably, the topical cosmetic (preferably skincare) compositions according to the invention comprise a thickener in particular if the cosmetic composition is in the form of an emulsion to assist in making the consistency of a product suitable. Preferred thickeners are aluminiumsilicates, xanthan gum, hydroxypropylmethylcellulose, hydroxyethylcellulose, polyacrylates such as carbopole^{®} (e.g., Carbopole 980, 981, 1382, 2984, 5984) or mixtures thereof. Further preferred thickeners encompass acrylate/C10-30 alkyl acrylate copolymers (such as e.g., Pemulen TR 1, Pemulen TR 2, Carbopol 1328 by. NOVEON) as well as AristoflexAVC (INCI: Ammonium Acryloyldimethyltaurate/VP Copolymer).

The topical cosmetic (preferably skincare) composition according to the present invention advantageously comprise a preservative. When present, the preservative is preferably used in an amount of 0.1 to 2 wt.-%, more preferably in an amount of 0.5 to 1.5 wt.-%, based on the total weight of the composition.

The topical cosmetic (preferably skincare) composition according to the invention in general have a pH in the range of 3 to 10, preferably a pH in the range of 4 to 8 and most preferably a pH in the range of 4 to 7.5 such as in the range of 5 to 6.5. The pH can easily be adjusted as desired with suitable acids, such as citric acid or bases, such as sodium hydroxide (e.g., as aqueous solution), triethanolamine (TEA Care), Tromethamine (Trizma Base), and Aminomethyl Propanol (AMP-Ultra PC 2000), according to standard methods in the art.

The amount of the topical cosmetic (preferably skincare) composition to be applied to the skin is not critical and can easily be adjusted by a person skilled in the art. Preferably the amount is selected in the range of 0.1 to 3 mg/cm² skin, such as preferably in the range of 0.1 to 2 mg/cm² skin and most preferably in the range of 0.5 to 2 mg/cm² skin.

In a specific embodiment, the present invention also relates to a method of treating skin ageing, said method comprising the step of (i) applying a skin care preparation with all the definition and preferences as given herein to the skin, which is in need of the treatment, preferably at least once a day for at least two days, more preferably at least once a day for at least 7 days, even more preferably at least once a day for at least 1 month and (ii) optionally appreciating the effect after the period of treatment.

In another particular embodiment, the topical cosmetic compositions are hair care preparations.

The final product form of the hair care preparation according to the invention may suitably be, for example, shampoos, conditioners, sprays, mousses, gels, oils, creams, waxes, hair tonics, hair essences, or lotions. Particularly preferred product forms are leave-in products, especially post-wash conditioners (leave-in) and hair treatment products such as hair tonics or essences. Particular preferred in all embodiments of the present invention are leave-in hair care preparations.

Conditioner compositions according to the present invention usually comprise one or more conditioning surfactants, which are cosmetically acceptable and suitable for topical application to the hair. Suitable conditioning surfactants are selected from cationic surfactants, used singly or in a mixture.

Cationic surfactants useful in hair care preparations according to the present invention contain amino or quaternary ammonium hydrophilic moieties, which are positively charged when, dissolved in the aqueous composition of the present invention.

The most preferred cationic surfactants for conditioner compositions according to the present invention are monoalkyl quaternary ammonium compounds in which the alkyl chain length is C₁₆ to C₂₂.

Examples of suitable cationic surfactants include quaternary ammonium compounds, particularly trimethyl quaternary compounds. Preferred quaternary ammonium compounds include cetyltrimethylammonium chloride, behenyltrimethylammonium chloride (BTAC), cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, PEG-2 oleylammonium chloride and salts of these where the chloride is replaced by halogen, (e.g., bromide), acetate, citrate, lactate, glycolate, phosphate nitrate, sulphate, or alkylsulphate. Further suitable cationic surfactants include those materials having the CTFA designations Quaternium-5, Quaternium-31 and Quaternium-18. Mixtures of any of the foregoing materials may also be suitable. A particularly useful cationic surfactant for use in hair conditioners according to the present invention is cetyltrimethylammonium chloride.

Salts of primary, secondary, and tertiary fatty amines are also suitable cationic surfactants.

In the conditioners according to the present invention, the level of cationic surfactant is preferably selected in the range from 0.01 to 10 wt.-%, more preferably from 0.05 to 5 wt.-%, most preferably from 0.1 to 2 wt.-%, based on the total weight of the composition.

Conditioner compositions according to the present invention preferably additionally comprise fatty materials. By "fatty material" is meant a fatty alcohol, an alkoxylated fatty alcohol, a fatty acid, a glyceride, glycerol, plant unsaponifiables or a mixture thereof. Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of the composition.

Ethoxylated fatty alcohols having from about 12 to about 18 carbon atoms in the alkyl chain can be used in place of, or in addition to, the fatty alcohols themselves. Suitable examples include ethylene glycol cetyl ether, polyoxyethylene (2) stearyl ether, polyoxyethylene (4) cetyl ether, and mixtures thereof.

The level of fatty alcohol material in conditioners according to the present invention is suitably selected in the range from 0.01 to 15 wt.-%, preferably from 0.1 to 10 wt.-%, and more preferably from 0.1 to 5 wt.-%, based on the total weight of the composition. The weight ratio of cationic surfactant to fatty alcohol is preferably selected in the range from 10:1 to 1:10, preferably from 4:1 to 1:8, most preferably from 1:1 to 1:7, such as for example 1:3.

In a preferred embodiment, the hair care preparations, especially if it is a shampoo, further comprises from 0.1 to 5 wt.-%, based on the total weight of the composition of a suspending agent.

Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives, and mixtures thereof. The long chain acyl derivative is desirably selected from ethylene glycol stearate, alkanolamides of fatty acids having from 16 to 22 carbon atoms and mixtures thereof. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich. Suitable cross-linked polymers of acrylic acid and acrylate esters are Pemulen TR1 or Pemulen TR2 (both available from Lubrizol). A suitable heteropolysaccharide gum is xanthan gum.

The hair care preparations according to the present invention may further contain emulsified droplets of a silicone-conditioning agent for enhancing conditioning performance. Suitable silicones include polydiorganosiloxanes, in particular polydimethylsiloxanes which have the CTFA designation dimethicone. Also suitable for use compositions according to the present invention (particularly shampoos and conditioners) are polydimethyl siloxanes having hydroxyl end groups, which have the CTFA designation dimethiconol. Also suitable for use in compositions according to the present invention are silicone gums having a slight degree of cross-linking.

Examples of suitable pre-formed silicone emulsions include dimethiconol emulsions DC2-1766, DC2-1784, DC-1785, DC-1786 and DC-1788, all available from Dow Corning. Also suitable are amodimethicone emulsions such as DC2-8177 and DC939 (from Dow Corning) and SME253 (from GE Silicones).

A further preferred class of silicones for inclusion in shampoos and conditioners of the present invention have at least one amino functional group.

The total amount of silicone is preferably selected in the range from 0.01 to 10 wt.-%, more preferably from 0.3 to 5 wt.-%, most preferably 0.5 to 3 wt.-%, based on the total weight of the composition.

The hair care preparations according to the present invention may also comprise a dispersed, non-volatile, water-insoluble oily conditioning agent.

By "insoluble" is meant that the material is not soluble in water (distilled or equivalent) at a concentration of 0.1 percent w/w at 25 degrees centigrade Suitable oily or fatty materials are selected from hydrocarbon oils, fatty esters and mixtures thereof. Adjuvants

The hair care preparations of the present invention may also contain adjuvants suitable for hair care. Generally, such ingredients are included individually at a level of up to 2 wt.-%, preferably up to 1 wt.-%, based on the total weight of the composition.

Suitable hair care adjuvants, include amino acids, sugars and ceramides. Particularly preferred are anti-dandruff agents, especially those comprising zinc, such as zinc pyrithione (ZnPTO). A further preferred ingredient is climbazole.

The hair styling polymer, if present, is preferably present in the hair care compositions according to the present invention in an amount of from 0.001 to 10 wt.-%, more preferably from 0.1 to 10 wt.-%, such as from 1 to 8 wt.-%, based on the total weight of the composition. Hair styling polymers are well known. Suitable hair styling polymers include commercially available polymers that contain moieties that render the polymers cationic, anionic, amphoteric or nonionic in nature. Suitable hair styling polymers include, for example, block and graft copolymers. The polymers may be synthetic or naturally derived.

Shampoo compositions preferably comprise one or more cleansing surfactants, which are cosmetically acceptable and suitable for topical application to the hair. Further surfactants may be present as emulsifiers.

Suitable cleansing surfactants are selected from anionic, amphoteric and zwitterionic surfactants and mixtures thereof. The cleansing surfactant may be the same surfactant as the emulsifier or may be different. Anionic Cleansing Surfactant

Shampoo compositions according to the invention will typically comprise one or more anionic cleansing surfactants, which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates and alpha-olefin sulphonates, especially their sodium, magnesium, ammonium and mono-, di- and triethanolamine salts.

Typical anionic cleansing surfactants for use in shampoo compositions according to the present invention include sodium oleyl sulpho succinate, ammonium lauryl sulphosuccinate, ammonium lauryl sulphate, sodium cocoyl isethionate, sodium lauryl isethionate and sodium N-lauryl sarcosinate. The most preferred anionic surfactants are sodium lauryl sulphate, sodium lauryl ether sulphate (n)EO (where n ranges from 1 to 3), ammonium lauryl sulphate and ammonium lauryl ether sulphate (n)EO (where n ranges from 1 to 3).

The total amount of anionic cleansing surfactant in shampoo compositions according to the present invention is generally from 5 to 30 wt.-%, preferably from 6 to 20 wt.-%, more preferably from 8 to 16 wt.-%, based on the total weight of the composition.

The shampoo composition may also include co-surfactants, preferably an amphoteric or zwitterionic surfactant, which can be included in an amount ranging from 0.01 to about 8 wt.-%, preferably from 1 to 4 wt.-%, based on the total weight of the composition.

Examples of amphoteric and zwitterionic surfactants include, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoo compositions according to the present invention include lauryl amine oxide, cocodi methyl sulphopropyl betaine and preferably lauryl betaine, cocamidopropyl betaine and sodium cocamphopropionate.

Another preferred co-surfactant is a nonionic surfactant, which can be included in an amount ranging from 0.01 to 8 wt.-%, preferably from 2 to 5 wt.-%, based on the total weight of the composition.

For example, representative nonionic surfactants that can be included in shampoo compositions according to the present invention include condensation products of aliphatic (C₈ - C₁₈) primary or secondary linear or branched chain alcohols or phenols with alkylene oxides, usually ethylene oxide and generally having from 6 to 30 ethylene oxide groups.

Further nonionic surfactants which can be included in shampoo compositions according to the present invention are the alkyl polyglycosides (APGs). Such APG's are well known to a person skilled in the art and e.g. commercially available as Plantacare UP 2000.

The shampoo composition can also optionally include one or more cationic co- surfactants included in an amount ranging from 0.01 to 10 wt.-%, more preferably from 0.05 to 5 wt.-%, most preferably from 0.05 to 2 wt.-%, based on the total weight of the composition. Useful cationic surfactants are described hereinbelow in relation to conditioner compositions.

The total amount of surfactant (including any co-surfactant, and/or any emulsifier) in shampoo compositions according to the present invention is generally from 5 to 50 wt.-%, preferably from 5 to 30 wt.-%, more preferably from 10 to 25 wt.-%, based on the total weight of the composition.

A cationic surfactant is a preferred ingredient in compositions according to the present invention for enhancing conditioning performance. Suitable cationic conditioning surfactants include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (e.g., Arquad 2HT/75 from Akzo Nobel), cocotrimethylammonium chloride, PEG-2-oleammonium chloride and the corresponding hydroxides thereof, Quaternium-5, Quaternium-31, Quaternium-18 and mixtures thereof.

Another example of a class of suitable cationic conditioning surfactants either alone or in admixture with one or more other cationic conditioning surfactants, is a combination of an amidoamine and an acid. Preferred amidoamines useful herein include stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylmine, behenamido-ethyl-diethylamine, behenamidoethyldimethylamine, arachidamidopropyldimethylamine, arachidamidopropyldiethylamine, arachid- amidoethyldiethylamine, arachidamidoethyldimethylamine, and mixtures thereof. The acid may be any organic or mineral acid which is capable of protonating the amidoamine in the hair care composition thus forming a tertiary amine salt which is in effect is a non-permanent quaternary ammonium or pseudo-quaternary ammonium cationic surfactant. Suitable acids useful herein include hydrochloric acid, acetic acid, tartaric acid, fumaric acid, lactic acid, malic acid, succinic acid, and mixtures thereof. Suitably, the acid is included in a sufficient amount to protonate all the amidoamine present.

Cationic polymers are preferred ingredients for enhancing conditioning performance. Suitable cationic polymers may be homopolymers which are cationically substituted or may be formed from two or more types of monomers. The weight average (Mw) molecular weight of the polymers will generally be between 100 000 and 2 million Daltons. Cationic polymer will generally be present in a shampoo composition at levels of from 0.01 to 5 wt.-%, preferably from 0.05 to 1 wt.-%, more preferably from 0.08 to 0.5 wt.-%, based on the total weight of the composition. Suitable cationic polymers include, for example, cationic polysaccharide polymers, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C₁-C₇ alkyl groups, more preferably C₁-₃ alkyl groups. Other suitable spacers include vinyl esters, vinyl alcohol, maleic anhydride, propylene glycol and ethylene glycol.

The cationic surfactant or polymer will generally be present in compositions according to the present invention at levels of from 0.01 to 5 wt.-%, preferably from 0.05 to 1 wt.-%, more preferably from 0.08 to 0.5 wt.-%, based on the total weight of the composition. The combined use of fatty alcohols and cationic surfactants in conditioning compositions is believed to be especially advantageous, because this leads to the formation of a lamellar phase, in which the cationic surfactant is dispersed. The weight ratio of cationic surfactant to fatty alcohol is suitably selected in the range from 1:1 to 1:10, preferably from 1:1.5 to 1:8, most preferably from 1:2 to 1:5. If the weight ratio of cationic surfactant to fatty alcohol is too high, this can lead to eye irritancy from the composition. If it is too low, it can make the hair feel squeaky for some consumers.

If the hair care preparation is a hair tonic said hair tonic can contain additional conventional additive ingredients that are commonly used in such tonics, such as perfume oils, in an amount of from 0.01 to 0.5 wt.-%; turbidity-inducing agents, such as ethylene glycol distearate, in an amount of about 0.2 to 5 wt.-%; surfactants, especially emulsifiers; solvating agents; preservatives, such as e.g. para-hydroxybenzoic acid, in an amount of from 0.01 to 1 wt.-%; buffer substances, such as sodium citrate or sodium phosphate, in an amount of from 0.1 to 1 wt.-%; care materials, such as hair-care-providing or skin-care-providing plant or vegetable extracts, protein hydrolysates and silk hydrolysates, lanolin derivatives, in an amount of from 0.1 to 5 wt.-%; physiologically compatible silicone derivative compounds, such as volatile or non-volatile silicone oils or high molecular weight siloxane polymers in an amount of from 0.05 to 20 wt.-%; light protecting agents, antioxidants, radical-trapping agents, anti-flaking agents, in an amount of about 0.01 to 2 wt.-%; hair luster-imparting agents, vitamins, combability improving agents and defatting agents.

The hair tonic according to the invention is employed according to a method in which the hair tonic is applied to the scalp in an amount sufficient to obtain the desired effect and is gently worked in.

The hair care preparations according to the present invention may contain other ingredients for enhancing performance and/or consumer acceptability. Such ingredients include fragrance, dyes and pigments, pH adjusting agents, pearlescence agents or opacifiers, viscosity modifiers, and preservatives or antimicrobials. Each of these ingredients will be present in an amount effective to accomplish its purpose. Generally, these optional ingredients are included individually at a level of up to 5 wt.-%, based on the total weight of the composition.

Hair care preparations according to the invention are produced using methods known to the person skilled in the art.

The amount of the hair care preparation to be applied to the scalp is not critical and can easily be adjusted by a person skilled in the art. Preferably the amount is selected in the range of 1 to 50ml, such as preferably in the range of 5 to 35 ml and most preferably in the range of 5 to 20 ml, per application.

In a specific embodiment, the present invention also relates to a method of treating hair ageing, said method comprising the step of (i) optionally wetting the hair with water, (ii) applying a hair care preparation with all the definition and preferences as given herein to the scalp (including the hair), (iii) allowing the scalp (including the hair) to stand for at least 30s, preferably 1 min, (iv) optionally rinsing off the compositions with water (e.g. in the case of a shampoo and/ or conditioner) and (v) further optionally appreciating the effect.

The following examples are provided to further illustrate the compositions and effects of the present invention. These examples are illustrative only and are not intended to limit the scope of the invention in any way.

### Examples

### 1. Cultivation of senescent cells

Normal and senescent cell populations were obtained from normal human dermal fibroblasts. For the normal cell population, the cells were seeded in cell culture medium (Dulbecco's Modified Eagle Medium supplemented with 10% serum and 1% antibiotics) and allowed to adhere and proliferate in a humidified cell incubator maintaining 37°C and 5% CO₂. These were then passaged or collected when 90% confluent. For the senescent cell population, the normal fibroblast cells were at this stage shortly exposed to 200 µM H₂O₂ followed by normal cell culture medium. Several days later, the cells were treated again with H₂O₂ to obtain a fully senescent population.

### 2. Senolytic activity study

Both normal and senescent populations were mixed into a 70% normal and 30% senescent cell population and represented the treatment population. Afterward, the 70% normal and 30% senescent cell population were seeded in cell culture medium and allowed to adhere in the cell incubator. After adherence, the medium was aspirated and the treatment medium (Dulbecco's Modified Eagle Medium supplemented with 1% serum) including myricitrin at various concentrations (see Table 1) added to the cells. For controls, there was one condition with treatment medium only without compound (negative control) and one condition with navitoclax, which is a known senolytic (positive control). The cells were incubated in the cell incubator for at least 72 hours. Afterward, senescence-associated beta-galactosidase was visualized - a well-known senescence marker - in the cells to quantify the senescence levels using flow cytometry. The results are depicted in table 1.

### Results

Experimentation showed that application of myricitrin to senescent cells had a significant senolytic effect and reduced the senescence percentage ratio versus normal cells.

**Table 1**

| Bioactive | Concentration | Senescent cells (%) | Standard deviation (%) | P-value | Significant senolytic effect |
|---|---|---|---|---|---|
| CTRL | | 29 | 3 | - | No |
| Navitoclax | 1.5 µM | 10 | 1 | <0.001 | Yes |
| Myricitrin | 25 µM | 13 | 2 | <0.001 | Yes |
| | 5 µM | 18 | 0 | <0.001 | Yes |
| | 1 µM | 24 | 1 | 0.006 | Yes |

Table 1. 70% normal and 30% senescent human dermal fibroblasts were treated for 72 hours with several concentrations of myricitrin, which resulted in the reduction of senescent cells. Data are normalized to the untreated control (30% senescent cells and 70% normal cells) and expressed as the mean ± SD (n = 4).

**Table 2**

| Bioactive | Concentration | Senescent cells (%) | Standard deviation (%) | P-value | Significant senolytic effect |
|---|---|---|---|---|---|
| CTRL | | 32 | 2 | - | No |
| Navitoclax | 3 µM | 18 | 1 | <0.001 | Yes |
| Myricitrin | 50 µM | 17 | 1 | <0.001 | Yes |
| | 10 µM | 19 | 1 | <0.002 | Yes |
| | 1 µM | 28 | 2 | <0.002 | Yes |

Table 2. 70% normal and 30% senescent hair follicle dermal papilla cells were treated for 72 hours with several concentrations of specific plant extracts, which resulted in the reduction of senescent cells for certain plant species. Data are normalized to the untreated control (30% senescent cells and 70% normal cells) and expressed as the mean ± SD (n = 4).

## Claims

1. Cosmetic use of myricitrin as an active ingredient for the treatment of the signs of skin or hair aging by selectively eliminating senescent skin or hair cells.

2. The use according to claim 1, wherein the senescent skin cells are human dermal fibroblasts and the hair cells are hair follicle dermal papilla cells.

3. The use according to claim 1 and/or 2, wherein the senescent cells are age-related senescent cells, stress-related senescent cells, or both.

4. The use according to anyone or more of claims 1 to 3, wherein the number of senescent cells is reduced by at least 10%.

5. The use according to anyone or more of claims 1 to 4, wherein the myricitrin is administered in the form of a cosmetic composition comprising an effective amount of myricitrin and a cosmetically acceptable carrier.

6. The use according to claim 5, wherein the carrier consists of at least 30 wt.-%, more preferably of at least 40 wt.-%, most preferably of at least 45 wt.-% of water, such as in particular of 50 to 90 wt.-% of water.

7. The use according to claim 5 and/or 6, wherein the cosmetic composition comprises from 0.001 to 10 wt.-%, preferably from 0.01 to 8 wt.-%, even more preferably from 0.1 to 7 wt.-%, furthermore preferably from 0.1 to 5 wt.-% and still more preferably from 0.1 to 3 wt.-%, of the myricitrin, based on the total weight of the cosmetic composition.

8. The use according to anyone or more of claims 5 to 7, wherein the cosmetic composition is a leave-on or rinse-off composition, preferably a leave-on composition.

9. The use according to anyone or more of claims 5 to 8, wherein, the composition is a skin care or a hair care preparation.

10. The use according to claim 9, wherein, the skin care preparation is an O/W emulsion comprising an oily phase dispersed in an aqueous phase.

11. The use according to claim 9, wherein, the hair care preparation is a shampoo, a conditioner, a spray, a mousse, a gel, an oil, a cream, a wax, a hair tonic, a hair essence, or lotion.

12. A cosmetic skin care method for selective removal of senescent cells in the skin, said method encompassing the step of applying, onto the skin in need, at least an effective amount of an extract of myricitrin; or a composition comprising at least said extract.

13. The method according to claim 12, wherein the senescent cells are senescent human dermal fibroblasts and/ or are hair follicle dermal papilla cells.

14. The method according to claim 12 and/or 13, wherein the effective amount of the myricitrin (based on dry matter) applied to the skin cells is selected in the range of 0.001 to 1 wt.-%/cm² of skin.

15. The method according to anyone of claims 12 to 14, wherein the number of senescent cells is reduced by at least 20%, preferably by at least 25%, most preferably by at least 30%.
